# EUROPEAN PATENT SPECIFICATION

(11) **EP 2 618 873 B1**
(45) Date of publication and mention of the grant of the patent: **11.03.2020**
(21) Application number: 11776590.9
(22) Date of filing: 21.09.2011
(51) Int. Cl.: A61M 5/32, A61B 1/303, A61B 1/32, A61M 29/00, A61M 37/00, A61M 25/00, A61M 5/34

(54) **SPECULUM WITH PLURALITY OF EXTENDABLE MULTI-DIRECTIONAL INJECTION NEEDLES**
SPEKULUM MIT MEHREREN AUSFAHRBAREN MULTI-DIREKTIONALEN INJEKTIONSNADELN
SPÉCULUM DOTÉ DE PLUSIEURS AIGUILLES D'INJÉCTION MULTI-DIRECTIONELLES EXTENSIBLES

(30) Priority: 21.09.2010 US 384717 P
(43) Date of publication of application: 31.07.2013
(73) Proprietor: Shamir Lebovitz, Israel, 67456 Tel-Aviv (IL)
(72) Inventor: Shamir Lebovitz, Israel, 67456 Tel-Aviv (IL)
(74) Representative: Lecomte & Partners
(86) International application number: PCT/IL2011/000746
(87) International publication number: WO 2012/038959

(56) References cited:
- EP-A1- 1 825 824
- EP-A1- 2 451 522
- WO-A1-2010/024871
- WO-A2-2007/079152
- WO-A2-2010/040556
- US-A- 5 419 777
- US-A1- 2002 031 513
- US-A1- 2005 261 662
- US-B1- 6 743 211

## Description

### FIELD OF THE INVENTION

The present invention generally relates to a substance delivery device and, more specifically, to a multi-directional needle assembly.

### BACKGROUND OF THE INVENTION

Hypodermic needles are hollow needles commonly used with a syringe to inject substances into the body or extract liquids from it. There are several medical situations where it is desired to deliver substances by injection to a relatively large volume of tissue. If a substance is delivered by a single point injection, the problems that may arise are: the substance cannot spread throughout the volume in sufficient time; too much dilution may occur during the spreading; the distribution of the substance within the volume may be very inhomogeneous; and, unwanted spreading to regions away from the target volume may occur. One solution to this problem is to give smaller injection at several sites within the target volume. This approach has at least three disadvantages: multiple needle stab wounds are created; accuracy of placement is limited; and the time for the procedure is increased. Therefore, multiple needle arrangements have been invented into to deliver the substance to the treatment area in an efficient manner.

An example of a multiple needle arrangement is disclosed in US patent 6,730,061. This patent discloses a hypodermic needle which comprises a first, hollow needle having movably secured therein one or more further, hollow needles. Each further needle and part of the hypodermic needle being movable relative to one another between a stressed position and an unstressed position. In the stressed position, each further needle is substantially parallel to the first needle. In the unstressed position, the free end of each further needle lies beyond the axial and/or radial terminus of the first needle.

Another example of a multiple needle arrangement is disclosed in US patent 6,302,870. This patent discloses an apparatus for injecting fluids into the walls of blood vessels, body cavities, and the like, and includes a plurality of laterally flexible needles disposed in a catheter for exit either out the distal end of the catheter or through corresponding side openings in the catheter. In the latter case, the terminal ends of the needles would be curved laterally, with each terminal end being positioned in a respective side opening so that when the needles were moved forwardly in the catheter, the terminal ends of the needles would move laterally out the respective openings to pierce a vessel or cavity wall adjacent to which the catheter was positioned. Hilts positioned near the terminal ends of the needles serve to control the depth of penetration of the needles.

A further example of a multiple needle arrangement is disclosed in US patent 6,432,092 in which a tissue mapping injection device suitable for use during a lymphatic breast mapping procedure is provided. The device includes a housing having an elongated body portion extending distally therefrom. A plunger is slidably positioned within the housing. A connector rod is secured to the forward end of the plunger and extends distally through the elongated body portion. The plunger and the connector rod define a fluid delivery channel. A plurality of needles are secured to the distal end of the connector rod. Each of the needles is constructed from a shape memory material and defines a fluid injection channel which communicates with the fluid delivery channel. The plunger is movable from a retracted position wherein the needles are positioned within the elongated body portion to an advanced position wherein the needles extend outwardly from the distal end of the elongated body portion.

The document US 5,419,777 A1 discloses a further example of a needle assembly. The needles extend through the whole catheter to supply different substances to the body. The catheter of such a needle assembly is particularly thick. Document US 2005/0261662 describes a lateral needle injection apparatus and document EP2451522 describes a fluid injection device suitable for various medical procedures.

The main limitation of the multiple needle arrangement known in the art is that their 3D structure is not specifically designed for predetermined treatment areas.

For example, these multiple needle arrangements do not have a 3D structure designed for treating the upper and the lower lips of the cervix.

### SUMMARY OF THE INVENTION

The invention relates to a multi-directional needle assembly as claimed in claim 1.

It is one object of the present invention to provide a multi-directional needle assembly for injecting a substance into at least one injection site of a patient's body. The multi-directional needle assembly comprises:
a. an elongated member having a distal end; said distal end having a plurality of openings disposed therein; and,
b. a plurality of needles disposable at least partially within said elongated member, said needles adapted to be reconfigured from a FOLDED configuration to a DEPLOYED configuration and vice versa; said FOLDED configuration being characterized by the position of said plurality of needles within said elongated member; said DEPLOYED configuration being characterized by the protrusion of said needles out of said plurality of openings;

The disposition of said openings in said distal end is provided according to a predetermined scattering pattern such that (i) at least two separated areas within said distal end are provided for injection of said substance into at least two different injection locations at said injection site; and, (ii) a DEAD AREA between said two separated areas is obtained, wherein at least one of the following is being held true;
(i) said DEAD AREA is characterized by a solid angle of at least about 1 steradian;
(ii) said DEAD AREA is characterized by a spreading angle in the range of 20 to 60 degrees from each other;
(iii) said DEAD AREA is characterized by a maximal length of at least 0.1 nanometer; or,
(iv) said DEAD AREA is characterized by a geometrical area of at least 0.1 nanometer²;
(v) said DEAD AREA is characterized by a cross sectional area of at least 0.1 nanometer²; or any combination thereof.

It is within the scope of the present invention that the DEAD INJECTION LOCATIONS are characterized by a geometrical characteristic selected from the group consisting of: a solid angle of at least about 1 steradian; a spreading angle in the range of about 20° to about 60° from each other; a maximal length of at least about 0.1 nanometer; a geometrical area of at least about 0.1 nanometer²; a cross sectional area of at least about 0.1 nanometer²; and any combination thereof.

It is another object of the present invention to provide the multi-directional needle assembly as defined above, wherein in said DEPLOYED configuration, said needles are adapted to form at least two injection surfaces at said at least two separated areas, each of said at least two injection surfaces is adapted to conform to the anatomical shape of said at least two injection locations.

It is another object of the present invention to provide the multi-directional needle assembly as defined above, wherein said at least two injection surfaces are adapted to mimic the anatomical shape of said at least two injection locations.

It is another object of the present invention to provide the multi-directional needle assembly as defined above, wherein each of said at least two injection surfaces is characterized by a predetermined spread angle ranging from about 100° to about 170°.

It is another object of the present invention to provide the multi-directional needle assembly as defined above, wherein each of said at least two injection surfaces is characterized by a predetermined spread angle ranging from about 150° to about 160°.

It is another object of the present invention to provide the multi-directional needle assembly as defined above, wherein said at least two injection surfaces formed by different lengths of said needles.

It is another object of the present invention to provide the multi-directional needle assembly as defined above, wherein said at least two separated areas are adapted to be used for simultaneous injection of said substance into two injection locations.

It is another object of the present invention to provide the multi-directional needle assembly as defined above, wherein said two injection locations are: the upper lip of the cervix and the lower lip of the cervix.

It is another object of the present invention to provide the multi-directional needle assembly as defined above, wherein the needles are characterized by lengths ranging from about 0.5mm to about 4 cm.

It is another object of the present invention to provide the multi-directional needle assembly as defined above, wherein the predetermined scattering pattern is selected from a group consisting of: arbitrary, well organized, and any combination thereof.

It is another object of the present invention to provide the multi-directional needle assembly as defined above, wherein the said needles are microneedles.

It is another object of the present invention to provide the multi-directional needle assembly as defined above, wherein said needles are 20 guage to about 35 gauge needle.

It is another object of the present invention to provide the multi-directional needle assembly as defined above, wherein at least one of the needles is one of a group of: nano-sized, micro-sized, milli-sized, or any combination thereof.

It is another object of the present invention to provide the multi-directional needle assembly as defined above, wherein the needles are characterized by widths ranging from about 0.5 micron to about 400 micron.

It is another object of the present invention to provide the multi-directional needle assembly as defined above, wherein the needles are characterized by widths ranging from about 100 nm to about 500 nm.

It is another object of the present invention to provide the multi-directional needle assembly as defined above, further comprising at least one spreading mechanism adapted to reconfigure the needles within the elongated member from a FOLDED configuration to a DEPLOYED configuration and vice versa.

It is another object of the present invention to provide the multi-directional needle assembly as defined above, wherein the spreading mechanism is adapted to protract the needles when the same are converted from the FOLDED configuration to the DEPLOYED configuration, and to retract the needles when the same are converted from the DEPLOYED configuration to the FOLDED configuration.

It is another object of the present invention to provide the multi-directional needle assembly as defined above, wherein the substance is for induction of labor.

It is another object of the present invention to provide the multi-directional needle assembly as defined above, wherein the substance is a cervical-ripening amount of a collagenase or any naturally stimulating collagenase.

It is another object of the present invention to provide the multi-directional needle assembly as defined above, wherein the substance further comprises material selected from a group consisting of: Interleukin 1, Interleukin 1 beta, Interleukin 6, Interleukin 8, tissue inhibitors metalloproteinase 1-2, tumor necrosis factor, NAC n -acetyl cysteine TIMP tissue inhibitor metalloproteinases 1-2, inhibition anti TNF antibodies anti IL 1 beta antibodies TIMP 1-2 Alfa 2 macroglobulin, alfa 2 macroglobulin IL-8 ETE IL 1beta antibodies TNF antibodies adapted to prevent and/or treat preterm labor.

It is another object of the present invention to provide the multi-directional needle assembly as defined above, wherein the needles are made of a flexible material adapted to provide bending of the needles when they are reconfigured from the FOLDED configuration to the DEPLOYED configuration.

It is another object of the present invention to provide the multi-directional needle assembly as defined above, wherein said elongated member is selected from a group consisting of: a needle, a catheter, a lumen, and any combination thereof.

It is another object of the present invention to provide the multi-directional needle assembly as defined above, wherein at least two of said needles are aligned and oriented at substantially the same specific angle.

It is another object of the present invention to provide the multi-directional needle assembly as defined above, wherein at least two of said needles are randomly oriented.

It is another object of the present invention to provide the multi-directional needle assembly as defined above, wherein at least two of said needles are characterized by having the same length or thickness.

### BRIEF DESCRIPTION OF THE FIGURES

For a better understanding of the invention and to show how the same may be carried into effect, reference will now be made, purely by way of example, to the accompanying drawings in which like numerals designate corresponding elements or sections throughout.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the accompanying drawings:
- **FIG. 1**: is a schematic illustration of the multi-directional needle assembly of the present invention.
- **FIG. 2**: is an illustration of the cervix treated by the multi-directional needle assembly of the present invention.
- **FIG. 3**: is an illustration of the speculum with a plurality of needles disposed therein, and which speculum is not covered by the claims.
- **FIG. 4**: is an illustration of the cervix treated by the speculum with the plurality of needles.
- **FIGs. 5-12**: are illustrations of the speculum with a plurality of needles disposed therein, and which speculum is not covered by the claims.
- **Fig. 13-19**: are illustrations of another embodiment of the speculum with a plurality of needles disposed therein, and which speculum is not covered by the claims.

The drawings together with the description make apparent to those skilled in the art how the invention may be embodied in practice.

### DETAILED DESCRIPTION OF THE PREFERRED EMBODIMENTS

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

The present invention discloses a multi-directional needle assembly for injecting substance into at least one injection site of a body. According to the invention, the injection of the substance may be performed into two injection locations simultaneously.

The present invention discloses a multi-directional needle assembly for injecting substance into at least one injection site of a body, comprising: an elongated member having a distal end and a plurality of openings disposed therein; and, a plurality of needles located within the elongated member, the needles adapted to move within the elongated member and to be reconfigured from a FOLDED configuration to a DEPLOYED configuration and vice versa; the FOLDED configuration is characterized by the position of the plurality of needles being located within the elongated member; the DEPLOYED configuration is characterized by the protrusion of the needles out of the plurality of openings.

The disposition of said openings in said distal end is provided according to a predetermined scattering pattern such that (i) at least two separated areas within said distal end are provided for injection of said substance into at least two different injection locations at said injection site; and, (ii) a DEAD AREA between said two separated areas is obtained, wherein at least one of the following is being held true:
(i) (a) said DEAD AREA is characterized by a solid angle of at least at least about 1 steradian;
(ii) said DEAD AREA is characterized by a spreading angle in the range of 20 to 60 degrees from each other;
(iii) said DEAD AREA is characterized by a maximal length of at least 0.1 nanometer; or,
(iv) said DEAD AREA is characterized by a geometrical area of at least 0.1 nanometer²;
(v) said DEAD AREA is characterized by a cross sectional area of at least 0.1 nanometer²; or any combination thereof.

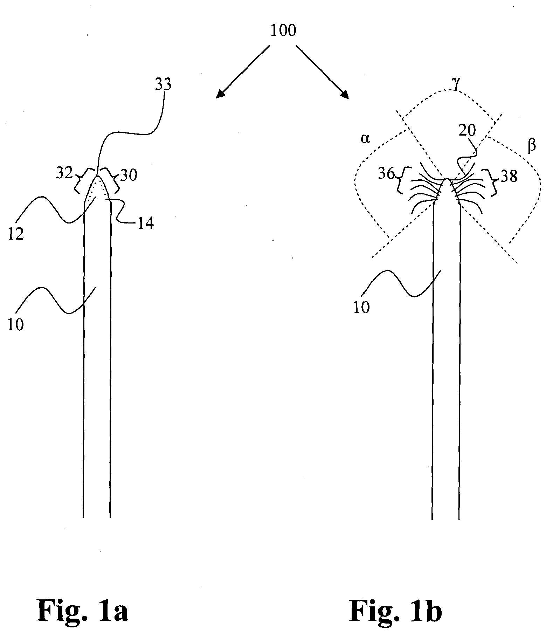

The term **'spread angle'** refers hereinafter to the angle which is able to define the size of a geometrical structure. The spread angle is an angle between two straight lines which exit from the same central point. For example, the angles α, β, or γ in Fig. 1b are all spread angles.

The term **'injection location'** refers hereinafter to a predetermined location which is part of an injection site, and to which a treatment by the device of the present invention may be provided.

The term **'DEAD AREA'** refers hereinafter to a predetermined area one the distal end of the device of the present invention, in which there are no openings which may be used for passage of needles. Therefore, there will be no injection of a substance to the corresponding body cavity.

The term **'DEAD INJECTION LOCATION'** refers hereinafter to a predetermined location at the injection site to which a treatment by the device of the present invention is NOT provided.

The term **'separated area'** refers hereinafter to a predetermined area at the device of the present invention, in which there are opening which may be used for passage of needles. The separated area may be characterized by a predetermined area or another geometrical characteristic which defines it and separates it from another area within the device.

The term **'injection surface'** refers hereinafter to a surface which may be formed by the ends of a plurality of needles. The 'injection surface' may be formed by extrapolation in a 2D/3D space of the ends of the needles.

The term **'scattering pattern'** refers hereinafter to a predetermined pattern of scattering of opening on a predetermined surface. The 'scattering pattern' may be used for determining the location of needles on an injection surface.

The term **'maximal length'** is a length of a straight line between two extreme points of a geometrical structure.

The term **'injection location'** refers hereinafter to a predetermined location at the injection site which may be characterized by a predetermined geometrical characteristic such as geometrical area.

The term **'solid angle'** refers hereinafter to the two-dimensional angle in three-dimensional space that an object subtends at a point. It is a measure of how large that object appears to an observer looking from that point. An object's solid angle is equal to the area of the segment of unit sphere (centered at the vertex of the angle) restricted by the object (this definition works in any dimension, including 1D and 2D). A solid angle equals the area of a segment of unit sphere in the same way a planar angle equals the length of an arc of unit circle. Solid angles can also be measured in square degrees (1 sr = (180/π)2 square degree) or in fractions of the sphere (i.e., fractional area), 1 sr = 1/4π fractional area.

The term **'protract'** refers hereinafter to pushing, thrusting, or extending (a part, etc.) outwards, especially a needle from within a predetermined region.

With reference to Figs. 1 to 4, numbered items are numbered consistently in these Figures so that, for example, the multi-directional needle assembly is numbered 100 in all said Figures.

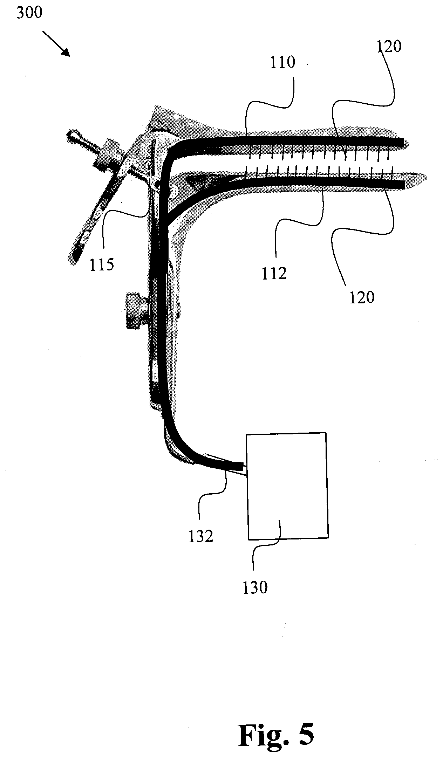

With reference to Figs. 5 to 19, numbered items are numbered consistently in these Figures so that, for example, the speculum is numbered 300 in all said Figures.

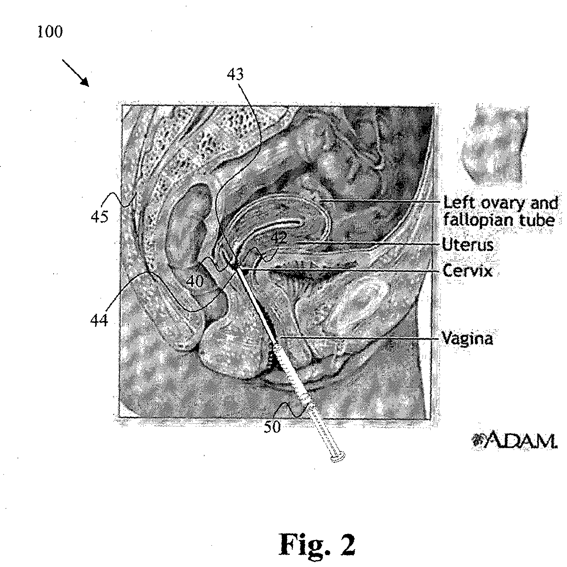

Reference is now made to Fig. 1a-b and Fig. 2 which schematically illustrate one embodiment of the present invention. According to this embodiment, a multi-directional needle assembly (100) for injecting substance into at least one injection site (44) of a patient's body (45) is disclosed. According to certain embodiments, the multi-directional needle assembly (100) may be connected to a syringe (50) in which the substance may be stored.

According to one embodiment, the multi-directional needle assembly (100) comprises the following elements:
a. An elongated member (10) having a distal end (12). The distal end (12) has a plurality of openings disposed therein.
b. A plurality of needles (20) located within the elongated member (10). The needles (20) are adapted to be reconfigured from a FOLDED configuration (Fig. 1a) to a DEPLOYED configuration (Fig. 1b) and vice versa.

The disposition of said openings in said distal end (12) is according to a predetermined scattering pattern such that: (i) at least two separated areas (30 and 32) within said distal end (12) for injection of the substance into at least two different injection locations (40 and 42) at the injection site (44) are provided; and, (ii) a DEAD AREA (33) between the two separated areas (30 and 32) is obtained, such that DEAD INJECTION LOCATIONS (43) at the injection site (44) are provided.

According to different embodiments of the present invention, DEAD INJECTION LOCATIONS (43) are characterized by a geometrical characteristic selected from the group consisting of: a solid angle of at least about 1 steradian; a spreading angle γ in the range of about 20° to about 60° from each other; a maximal length of at least about 0.1 nanometer; a geometrical area of at least about 0.1 nanometer²; a cross sectional area of at least about 0.1 nanometer²; or any combination thereof.

According to different embodiments, the predetermined scattering pattern may be arbitrary, well organized, or any combination thereof.

The FOLDED configuration which is schematically illustrated in Fig. 1a is characterized by the position of the needles (20) within the elongated member (10).

The DEPLOYED configuration which is schematically illustrated in Fig. 1b is characterized by the protrusion of the needles (20) out of the plurality of openings (14).

According to different embodiments of the present invention, the elongated member (10) may be a needle, a catheter, a lumen, or any type of member which may contain needles (20) and transport them to the treatment area.

According to different embodiments of the present invention, the substance may be any known in the art substance which is applicable to the human body.

According to the specific embodiment of Fig. 2, the two injection locations are: the upper lip of the cervix and the lower lip of the cervix. According to different embodiments of the present invention, the DEAD INJECTION LOCATION is the location between the upper and the lower lips of the cervix, a location where substance should not be injected. The device of the present invention is designed in a way which provides accurate injection of the substance to specific injection locations, while avoiding other locations which should not be treated.

According to this embodiment, the injected substance may be used for induction of labor. The substance may comprise a cervical-ripening amount of a collagenase or any naturally stimulating collagenase. The substance may further comprise material selected from the group consisting of: Interleukin 1 beta, Interleukin 6, Interleukin 8, tissue inhibitors metalloproteinase 1-2, tumor necrosis factor, NAC n -acetyl cysteine TIMP tissue inhibitor metalloproteinases1-2, inhibition anti TNF antibodies anti IL 1 beta antibodies TIMP 1-2 Alfa 2 macroglobulin, alfa 2 macroglobulin IL-8 ETE IL 1beta antibodies TNF antibodies adapted to prevent and/or treat preterm labor. The substance used by the present invention may also be the substance which is disclosed in patent 5,993,810. According to the embodiment in which the substance is used for inducing labor in a female, the two separated areas (30 and 32) are adapted to be used for simultaneous injection of the substance into two injection locations (40 and 42). The simultaneous injection of the substance into these two separated areas (30 and 32) may be important for providing a simultaneous effect in the upper and the lower lip of the cervix for softening or ripening the cervix. The cervix may be the uterine cervix of female mammals, including humans. According to the embodiments in which the substance comprises a collagenase or any naturally stimulating collagenase, it may accelerate labor, thus providing a superior efficacy compared to currently other substances.

Dealing with both the collagen matrix that is central in the cervical flexibility, and with other factors associated with rupture of membranes and uterine contractions, it provides a more efficient and reliable solution to the preterm labor problem and is far more effective than any of the other treatments and products that are in use today. Furthermore, this substance has potentially fewer side effects than other drugs. During in vitro feasibility studies, collagenase inhibition had achieved a 90% level potency. This substance may decrease the long-term healthcare expenses of babies, since they will be born more mature, have lower rates of morbidity, and will require less expenditure on long-term treatments and follow-up.

The present invention thus provides for the use of collagenase or any naturally stimulating collagenase and/or one or more substances which stimulate the production of naturally occurring collagen in obstetrics and gynecology to soften and ripen the cervix prior to termination of pregnancy or induction of labor in situations where the cervix is not in a favorable condition. Conventional procedures in which mechanical dilation of the cervix is effected by dilators with increasing diameters, can cause tearing or damage to the cervix. Induction of labor with prostaglandins locally and oxytocin intravenously can fail if the cervix is not in a favorable condition and may also be hazardous or toxic to the female if large doses are required. An advantage of using collagenase is that it comprises a naturally occurring enzyme that is physiologically compatible with the female's biochemistry and is generally non-toxic if used in prescribed dosages. The use of collagenase will facilitate induction of labor and termination of pregnancy and other procedures such as curettage, and is expected to minimize the incidence of caesarian sections which heretofore have needed to be performed. The use of collagenase is also expected to minimize or reduce damage to the uterine cervix caused during abortions using Hegar dilators, and to reduce cervix incompetence caused by any damage to the cervix during such operations.

According to certain embodiments, in the DEPLOYED configuration, the needles (20) are adapted to form two injection surfaces (36 and 38) at two separated areas (30 and 32). Each of the two injection surfaces (36 and 38) is adapted to conform to the anatomical shape of its corresponding injection locations (40 and 42). This conformation to the anatomical shape of the injection locations (40 and 42) may be expressed by mimicking the anatomical shape of the two injection locations.

According to different embodiments of the present invention, each of the injection surfaces (36 and 38) is characterized by a predetermined spread angle. According to some embodiments, the spread angle may be between about 100° and about 170°. According to other embodiments, the spread angle may be between about 150° and about 160°.

In Fig. 1b is illustrated two spread angles α and β. According to this embodiment the values of these angles are: α is about 155° and β is about 155°.

According to some embodiments, the injection surfaces (36 and 38) may be formed from different lengths of the needles. For example, the needles may be characterized by lengths which range from about 0.5 mm to about 4 cm.

According to some embodiments of the present invention, the needles may be microneedles. According to other embodiments, the needles may be nanoneedles. The terms 'microneedle' and 'nanoneedle' refer to any needle known in the art which has dimensions on either the micro scale or the nano scale.

According to some embodiments of the present invention, the needles may be characterized by a width between about 0.5 micron and about 400 micron. According to other embodiments, the needles may be characterized by a width between about 100 nm and about 500 nm.

According to some embodiments, the multi-directional needle assembly (100) may further comprise at least one spreading mechanism adapted to reconfigure the needles within the elongated member from a FOLDED configuration to a DEPLOYED configuration and vice versa. According to different embodiments, the spreading mechanism may protract the needles when the same are converted from the FOLDED configuration to the DEPLOYED configuration, and may retract the needles when the same are converted from the DEPLOYED configuration to the FOLDED configuration.

According to a different embodiment of the present invention, the needles may be made of a flexible material adapted to provide bending of the needles when the same are reconfigured from the FOLDED configuration to the DEPLOYED configuration.

According to different embodiments of the present invention, the structure of the distal end of the elongated member may be determined according to the anatomical structure of the treatment area. For example, according to one embodiment, the distal end of the elongated member may be characterized by a shape which is adapted to comply with and mimic the anatomical shape of the treatment area. In this embodiment, the needles may be characterized by all being the same length.

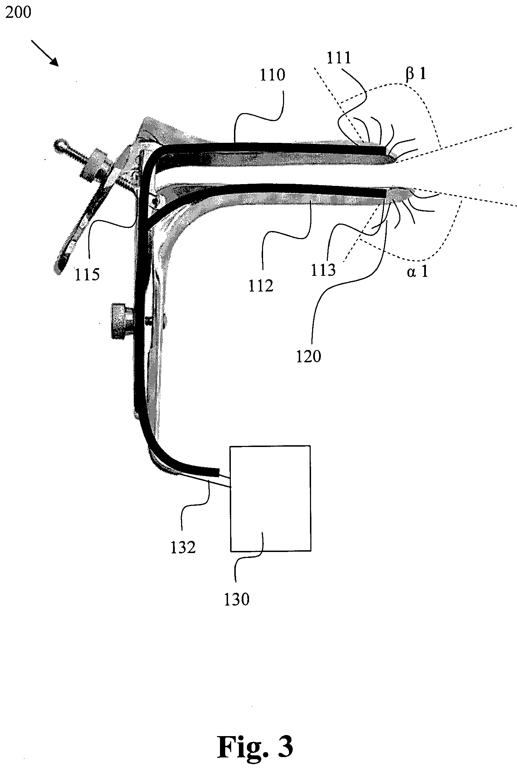

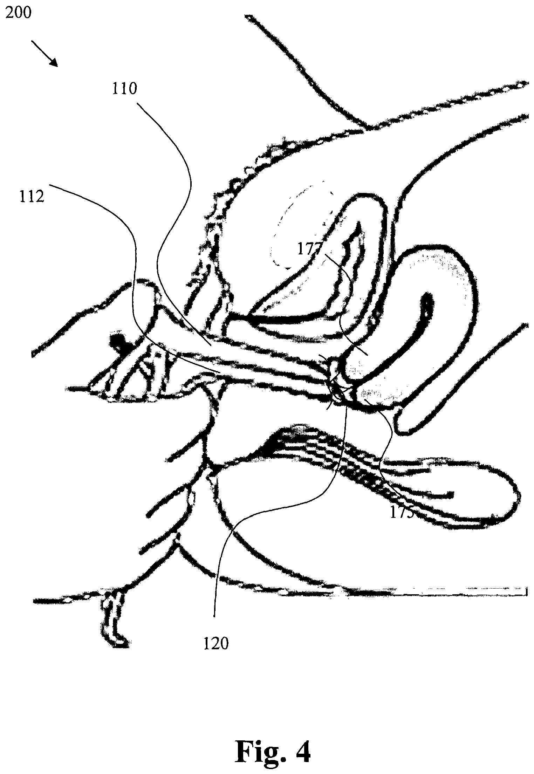

Reference is now made to Figs. 3 and 4 which illustrate a speculum. The speculum (200) is any speculum known in the art which, according to the present invention, has a plurality of needles disposed in its distal end. The speculum of the present invention is adapted to deliver a substance to the upper and the lower lips (175 and 177) of the cervix.

According to this embodiment, the speculum (200) comprises a first blade (110) with a distal end (111) and a second blade (112) with a distal end (113). The first blade (110) and second blade (112) are pivotally connected to each other by a connecting mechanism (115).

According to this embodiment, the distal end (111) of the first blade (112) and the distal end (113) of the second blade (116) have a plurality of openings disposed therein. The openings are adapted to accommodate a plurality of needles (120) which may be reconfigured from a FOLDED configuration to a DEPLOYED configuration and vice versa. The FOLDED configuration (not shown) is characterized by the position of needles (120) within the first and second blades (110 and 112). The DEPLOYED configuration (shown in Figs. 3 and 4) is characterized by the protrusion of needles (120) out of the plurality of openings of the first and second blades (110 and 112). Needles (120) of the first and said second blades (110 and 112) are fluidly connected via a supply line (132) to a substance reservoir (130) and are adapted to deliver the substance to the upper and the lower lips (175 and 177), respectively, of the cervix.

According to some embodiments, the needles are aligned and all the needles are positioned at a specific angle.

According to some embodiments, the needles are randomly oriented, or are oriented at a plurality of predetermined angles.

According to some embodiments, all the needles have the same length or thickness.

According to some embodiments, the needles have a plurality of different lengths and thicknesses; at least one needle has each of the predetermined combinations of length and thickness.

According to some embodiments, the needles are disposed along the entire length of the blades.

According to some embodiments, the needles are disposed in at least one specific region along the blades.

According to some embodiments, the disposition of the openings in the distal end is provided according to a predetermined scattering pattern.

According to some embodiments, in the DEPLOYED configuration, the needles of the first and the second blades are adapted to form two separate injection surfaces for conforming to the anatomical shape of the upper and lower lips of the cervix.

According to some embodiments, the injection surfaces are adapted to mimic the anatomical shape of the upper and lower lips of the cervix.

According to some embodiments, each of the injection surfaces is characterized by a predetermined spread angle ranging from about 100° to about 170°.

According to some embodiments, each of the injection surfaces is characterized by a predetermined spread angle ranging from about 150° to about 160°.

According to some embodiments, the injection surfaces are formed by different lengths of the needles.

According to some embodiments, the injection surfaces are characterized by a maximal length between about 0.5 cm to about 4 cm.

According to some embodiments, the needles are adapted to be used for simultaneous injection of the substance into the upper and lower lips of the cervix.

According to some embodiments, the needles are characterized by lengths ranging from about 0.5 mm to about 4 cm.

According to some embodiments, the predetermined scattering pattern is selected from a group consisting of: arbitrary, well organized, and any combination thereof.

According to some embodiments, at least one of the needles is one of a group of: nano-sized, micro-sized, milli-sized, or any combination thereof.

According to some embodiments, the needles are characterized by a width between about 0.5 micron to about 400 micron.

According to some embodiments, the needles are characterized by widths ranging from about 100 nm to about 500 nm.

According to some embodiments, the needles are 20 guage to about 35 gauge needle.

According to some embodiments, the speculum further comprises at least one spreading mechanism adapted to reconfigure the needles within the elongated member from a FOLDED configuration to a DEPLOYED configuration and vice versa.

According to some embodiments, the spreading mechanism is adapted to protract the needles when the same are converted from the FOLDED configuration to the DEPLOYED configuration, and to retract the needles when the same are converted from the DEPLOYED configuration to the FOLDED configuration.

According to some embodiments, the substance is for induction of labor.

According to some embodiments, the substance is a cervical-ripening amount of a collagenase or any naturally stimulating collagenase.

According to some embodiments, the substance further comprises material selected from the group consisting of: Interleukin 1 beta, Interleukin 6, Interleukin 8, tissue inhibitors metalloproteinase 1-2, tumor necrosis factor, NAC n -acetyl cysteine TIMP tissue inhibitor metalloproteinasesl-2, inhibition anti TNF antibodies anti IL 1 beta antibodies TIMP 1-2 Alfa 2 macroglobulin, alfa 2 macroglobulin IL-8 ETE IL 1beta antibodies TNF antibodies adapted to prevent and/or treat preterm labor.

According to some embodiments, the needles are made of a flexible material adapted to provide bending of the needles when the same are reconfigured from the FOLDED configuration to the DEPLOYED configuration.

According to some embodiments, the speculum comprises a first lumen adapted to deliver the substance from the substance reservoir to the needles.

Reference is now made to Figs. 5 to 12 which illustrate another embodiment. According to this embodiment, the speculum (300) is any speculum known in the art which, according to the present invention, comprises a plurality of needles disposed therein or thereupon.

The speculum is adapted to deliver a substance to the upper and lower lips of the cervix (175 and 177 illustrated in Fig. 4).

According to this embodiment, the speculum (300) comprises a first blade (110) and a second blade (112) each of which comprises an external surface and an internal surface. The first blade (110) and the second blade (112) are pivotally connected to each other by a connecting mechanism (115).

In the embodiments of Figs. 5 to 7 and Fig. 11, a plurality of needles (120) is coupled to the inward-facing surface of both of the blades (110 and 112).

In the embodiment of Fig. 5, the needles (120) are all the same length and thickness, and are aligned at the same angle.

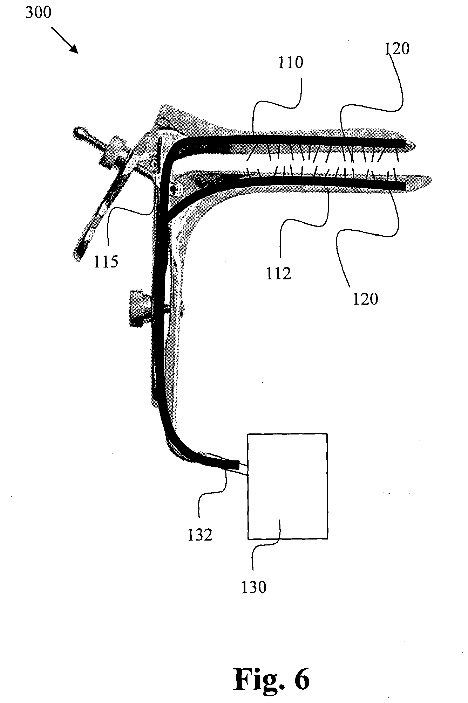

In the embodiment of Fig. 6, the needles (120) are all the same length and thickness, but are at different angles.

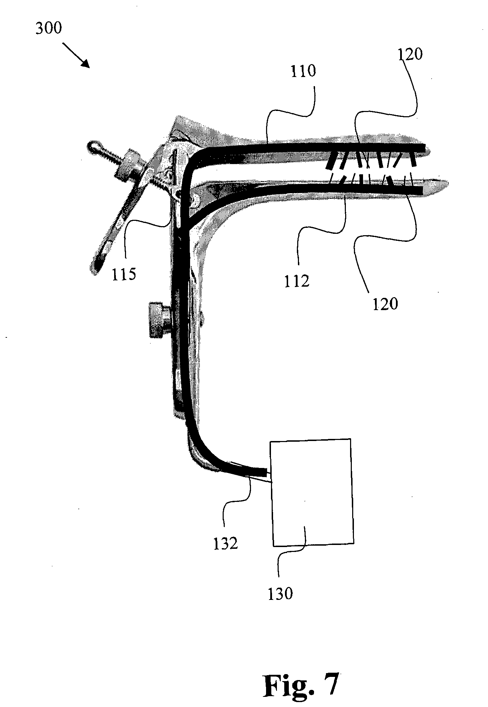

In the embodiment of Fig. 7, the needles (120) are of different lengths and thicknesses, and are at different angles.

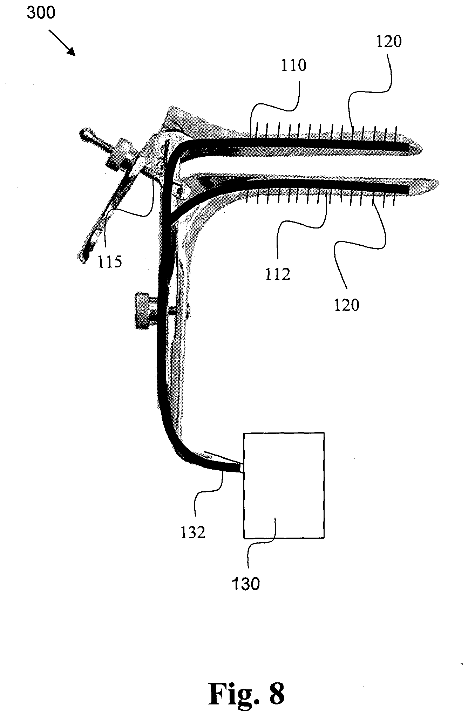

In the embodiment of Fig. 8, a plurality of needles (120) is coupled to the outward-facing surface of both of the blades (110 and 112).

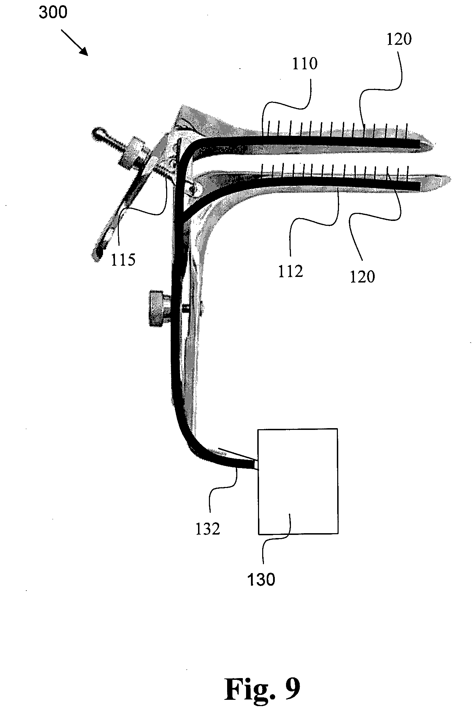

In the embodiment of Fig. 9, a plurality of needles (120) is coupled to the inward-facing surface of one of the blades (112) and to the outward-facing surface of the other blade (110).

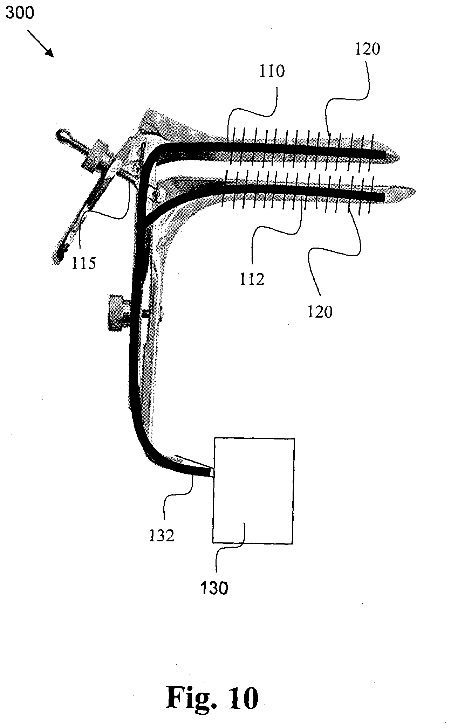

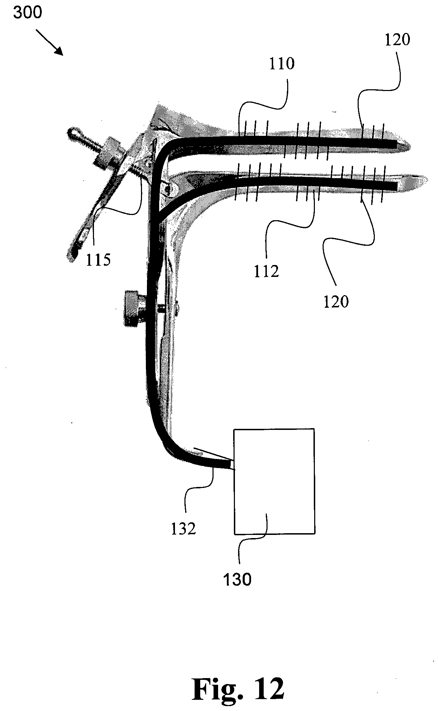

In the embodiment of Figs. 10 and 12, a plurality of needles (120) is coupled to both the inward-facing and the outward-facing surfaces of both of the blades (110 and 112).

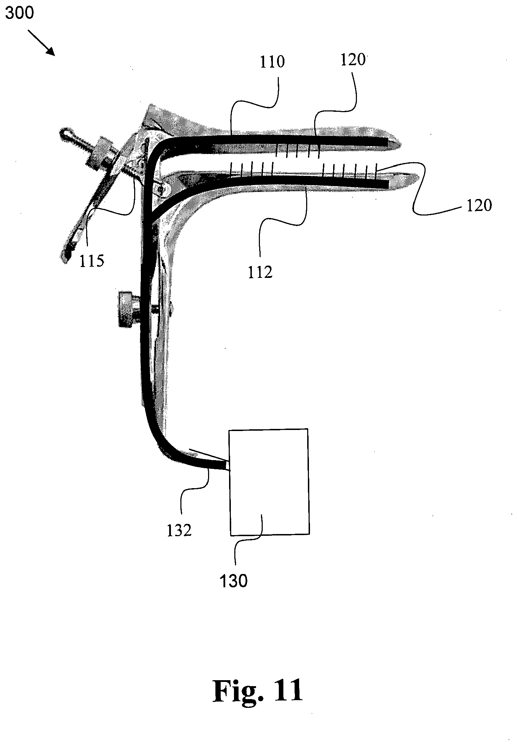

In the embodiment of Fig. 11, a plurality of needles (120) is coupled to the inward-facing surfaces of both of the blades at more than one region along the blade. In this embodiment, regions with needles on the lower blade (112) face regions without needles on the upper blade (110). Other dispositions of the needles will be obvious to one skilled in the art.

In the embodiment of Fig. 12, a plurality of needles (120) is coupled to both the inward-facing and the outward-facing surfaces of both of the blades. In this embodiment, regions with needles on the lower blade (112) face regions without needles on the upper blade (110). Similarly, on the outward-facing surfaces of the blades (110 and 112), regions with needles face regions without needles. In this embodiment, the needle patterns for the inward-facing surfaces of the blades differ from the needle patterns for the outward-facing surfaces of the blades. Other dispositions of the needles will be obvious to one skilled in the art.

According to one embodiment, the blades comprise a plurality of openings disposed therein, through which the needles are to protrude.

According to one embodiment, the needles are coupled to the internal surface of the blades; once the two blades are brought in proximity to each other, the needles in the internal surface of the blades are forced to protrude to the external surface (through said openings) and hence to deliver a substance to the cervix.

According to this embodiment, each of said blades comprises a plurality of openings disposed therein. The openings are adapted to accommodate a plurality of needles (120) which may be reconfigured from a FOLDED configuration to a DEPLOYED configuration and vice versa. The FOLDED configuration (not shown) is characterized by the position of the needles (120) within the first (110) and second blades (112).

The DEPLOYED configuration is characterized by the protrusion of needles (120) out of the plurality of openings in the first and second blades (110 and 112). The needles (120) of the first and said second blades (110 and 112), respectively, are fluidly connected via a supply line (132) to a substance reservoir (130) and adapted to deliver the substance to the cervix.

According to some embodiments, the needles are aligned and all the needles are positioned at a specific angle (see Fig. 5).

According to some embodiments, the needles are randomly oriented, or are oriented at a plurality of predetermined angles (see Fig. 6).

According to some embodiments, all the needles have the same length or thickness (see Figs. 5 or 6).

According to some embodiments, the needles have a plurality of different lengths and thicknesses; at least one needle has each of the predetermined combinations of length and thickness. (see Fig. 7).

According to some embodiments, the needles are disposed along the entire length of the blades (see. Figs. 5, 6 and 7).

According to some embodiments, the needles are disposed in at least one specific region along the blades (see. Figs. 4, 11 and 12).

According to some embodiments the disposition of the openings is provided according to a predetermined scattering pattern.

According to some embodiments, in the DEPLOYED configuration, the needles of the first and the second blades are adapted to form two separate injection surfaces for conforming to the anatomical shape of the cervix.

According to some embodiments, the injection surfaces are adapted to mimic the anatomical shape of the cervix.

According to some embodiments, each of the injection surfaces are characterized by a predetermined spread angle ranging from about 100° to about 170°.

According to some embodiments, each of the injection surfaces is characterized by a predetermined spread angle ranging from about 150° to about 160°.

According to some embodiments, the injection surfaces are formed by different lengths of the needles.

According to some embodiments, the injection surfaces are characterized by a maximal length of between about 0.5 cm to about 4 cm.

According to some embodiments, the needles are adapted to be used for simultaneous injection of the substance into the upper and lower lips of the cervix.

According to some embodiments, the needles are characterized by lengths ranging from about 0.5 mm to about 4 cm.

According to some embodiments, the predetermined scattering pattern is selected from a group consisting of: arbitrary, well organized, and any combination thereof.

According to some embodiments, at least one of the needles is one of a group of: nano-sized, micro-sized, or milli-sized.

According to some embodiments, the needles are characterized by a width of between about 0.5 micron to about 400 micron.

According to some embodiments, the needles are 20 guage to about 35 gauge needle.

According to some embodiments, the needles are characterized by widths ranging from about 100 nm to about 500 nm.

According to some embodiments, the speculum further comprises at least one spreading mechanism adapted to reconfigure the needles within the elongated member from a FOLDED configuration to a DEPLOYED configuration and vice versa.

According to some embodiments, the spreading mechanism is adapted to protract the needles when the same are converted from the FOLDED configuration to the DEPLOYED configuration, and to retract the needles when the same are converted from the DEPLOYED configuration to the FOLDED configuration.

According to some embodiments, the substance is for induction of labor.

According to some embodiments, the substance is a cervical-ripening amount of a collagenase or any naturally stimulating collagenase.

According to some embodiments, the substance further comprises material selected from the group consisting of: Interleukin 1 beta, Interleukin 6, Interleukin 8, tissue inhibitors metalloproteinase 1-2, tumor necrosis factor, NAC n -acetyl cysteine TIMP tissue inhibitor metalloproteinases1-2, inhibition anti TNF antibodies anti IL 1 beta antibodies TIMP 1-2 Alfa 2 macroglobulin, alfa 2 macroglobulin IL-8 ETE IL 1beta antibodies TNF antibodies adapted to prevent and/or treat preterm labor.

According to some embodiments, the needles are made of a flexible material adapted to provide bending of the needles when the same are reconfigured from the FOLDED configuration to the DEPLOYED configuration.

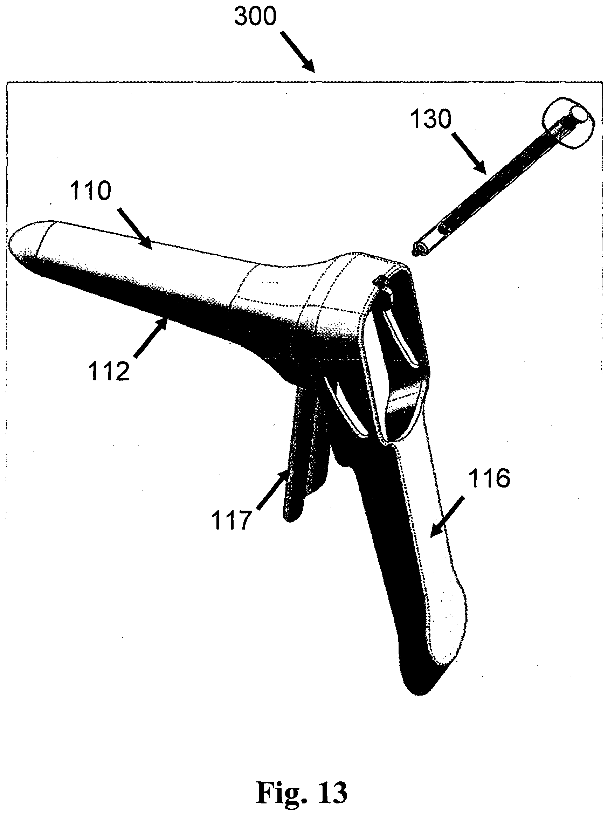

According to some embodiments, the speculum comprises a first lumen adapted to deliver the substance from the substance reservoir to the needles.Reference is now made to Figs. 13 to 19 which illustrate another embodiment of the present invention. According to this embodiment, the speculum (300) is any speculum known in the art which, according to the present invention, comprises a plurality of needles disposed therein or thereupon. The speculum of the present invention is adapted to deliver a substance to the upper and lower lips of the cervix (175 and 177 in Fig. 4).

According to this embodiment, the speculum (300) comprises a first blade (110) and a second blade (112) each of which comprises an external surface and an internal surface. The first blade (110) and the second blade (112) are pivotally connected to each other by a connecting mechanism (not shown). The speculum has a closing mechanism with a fixed handle part (116) connected to the upper blade (110) and a movable handle part (117) connected to the lower blade (112). It is also connected to a removable reservoir (130) which may also comprise a plunger for causing the substance to exit the reservoir and be delivered to the injection site. In Fig. 13, the reservoir (130) is shown separated from the speculum (300) for clarity.

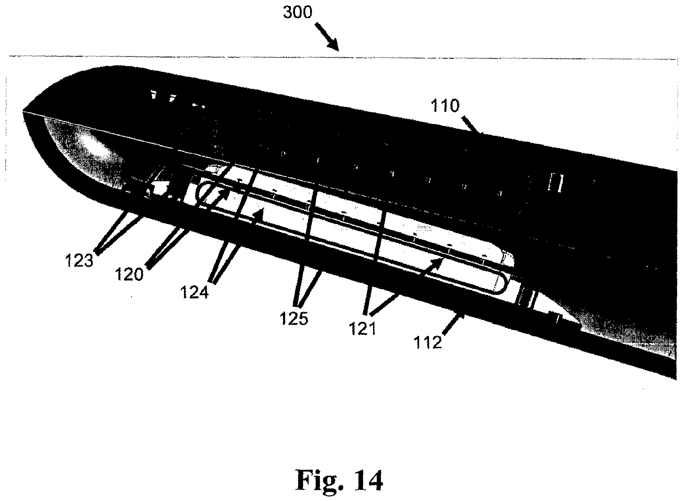

Reference is now made to Fig. 14. In Fig. 14, a side view is shown of a vertical section through the center of the speculum (300) of this embodiment. At least a portion of the inner surface of the upper blade (110) and the inner surface of the lower blade (112) comprises an injection mechanism. The injection mechanism comprises an outer plate (123), a container (124) and an inner bracket (125). The inner bracket is fastened, either permanently or removably, to the blade. The outer plate (123) of the injection mechanism has openings (121) for a plurality of needles (120), which are fluidly connected to the container (124). For clarity, only one needle (120) is shown.

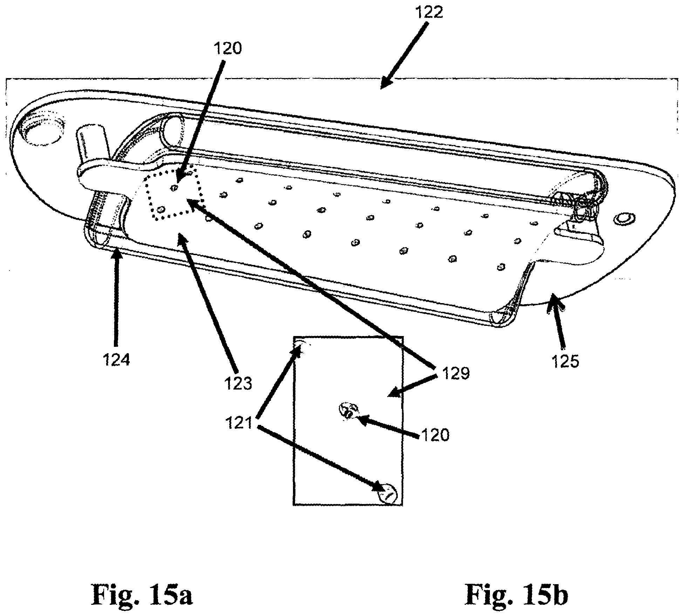

Reference is now made to Fig. 15. In Fig. 15, the speculum of this embodiment (300) is shown from two different angles, so that the injection mechanism may be seen in situ on the upper (Fig. 15a) and lower (Fig. 15b) blades. At least a portion of the inner surface of the upper blade (110) and the inner surface of the lower blade (112) comprises an injection mechanism. The injection mechanism comprises an outer plate (123), a container (124) and an inner bracket (125). The inner bracket is fastened, either permanently or removably, to the blade. The outer plate (123) of the injection mechanism has openings (121) for a plurality of needles (120), which are fluidly connected to the container (124). For clarity, only one needle (120) is shown.

In this embodiment, the upper blade (110) and the fixed handle (116) are connected to the lower blade (112) and the movable handle (117) by a slidable connecting mechanism (115).

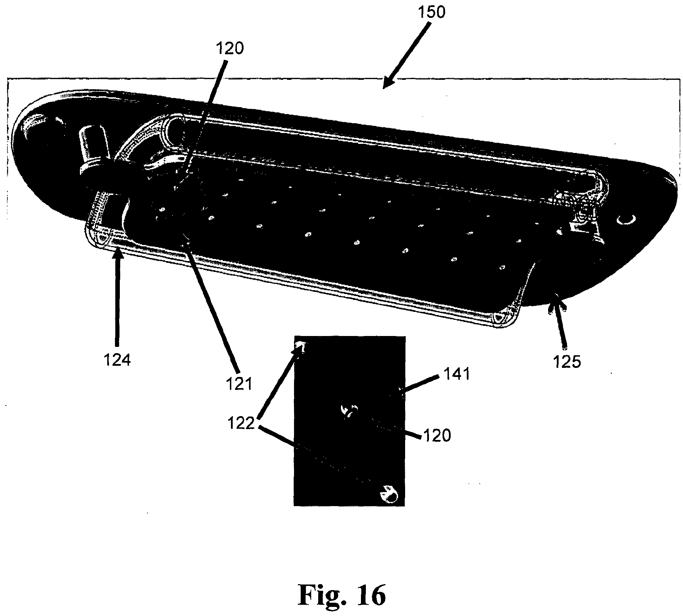

Reference is now made to Fig. 16. In Fig. 16, the injection mechanism (122) of this embodiment is shown. The inner bracket (125) is fastened, either permanently or removably, to a blade (not shown). Between the inner bracket (125) and the outer plate (123) is a container (124) which may hold within it at least a portion of the substance to be injected. The container (124) is fluidly connected, via a manifold and a tube (not shown) to a reservoir (not shown) and to a plurality of needles (120).

A region of the injection mechanism (129) of this embodiment is shown in close-up at the bottom of Fig. 16. In the close-up (129), as an illustrative example, a needle (120) is shown DEPLOYED in the central opening, while the needle is shown FOLDED in the other two openings (121).

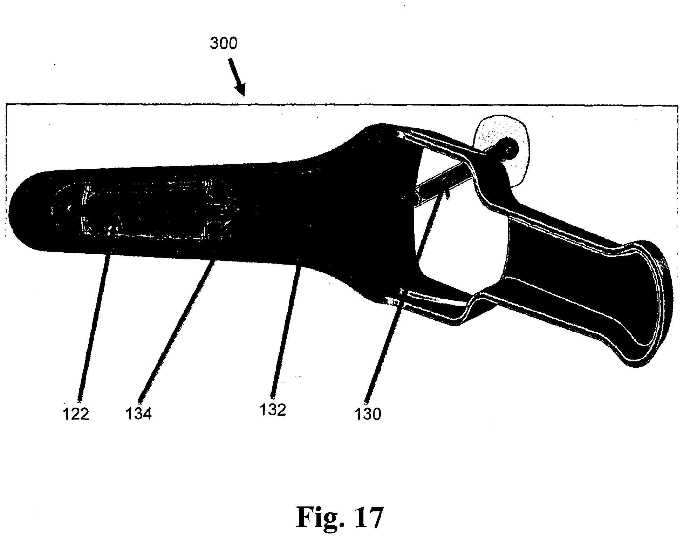

Reference is now made to Fig. 17. In Fig. 17 the connection between the reservoir (130) and the injection mechanism (122) is shown according to this embodiment of the speculum (300). In this embodiment, the connection comprises a silicone tube (132) fluidly connected at one end to the reservoir (130) and fluidly connected at the other end to the base of a "Y" manifold (134). Each of the arms of the "Y" manifold (134) is fluidly connected to the container (124), one to the left of center of the container, one to the right of center.

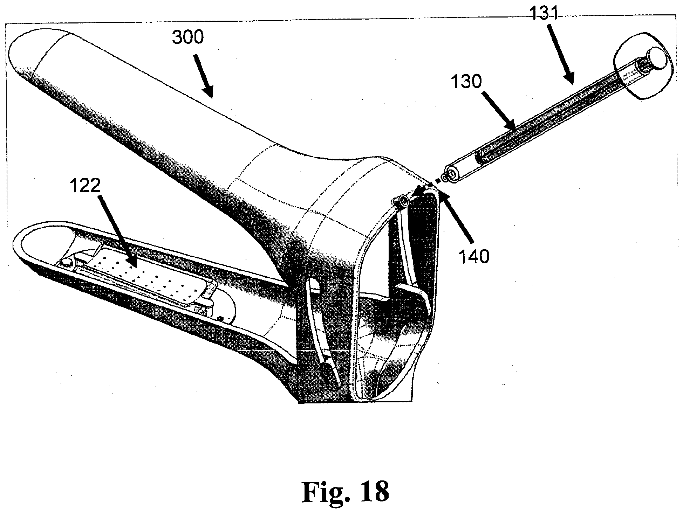

Reference is now made to Fig. 18. In this embodiment of the speculum (300), the reservoir (130) forms at least a portion of the interior of an injector (131). The position of the injector (131) just before it connects to the speculum (300) is shown. The dashed arrow (140) shows how the injector (131) connects to the speculum (300). For clarity, the connecting tubes are not shown.

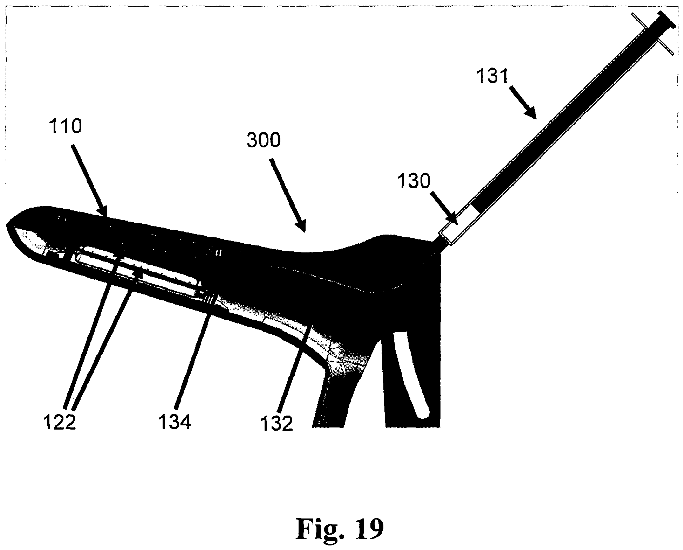

Reference is now made to Fig. 19, which shows a side view of a vertical section through the center of this embodiment of the speculum (300), showing the tubing connecting the injector (131) to the injection mechanism (122). The injector (131) comprising a fluid reservoir (130) is fluidly connected to one end of silicone tubing (132). The silicone tubing (132) is fluidly connected at its other end to the base of a "Y" manifold (134). The arms of the "Y" manifold (134) are fluidly connected to the injection mechanism (122). In Fig. 19, only the connection between the injector (131) and the injection mechanism on the upper blade (110) is shown for clarity.

## Claims

1. A multi-directional needle assembly (100) for injecting substance into at least one injection site of a patient's body, comprising:
a. an elongated member (10) having a distal end (12); said distal end (12) having a plurality of openings disposed therein; and,
b. a plurality of needles (20) disposable at least partially within said elongated member (10), said needles (20) adapted to be reconfigured from a FOLDED configuration to a DEPLOYED configuration and vice versa; said FOLDED configuration being **characterized by** the position of said plurality of needles (20) within said elongated member (10); said DEPLOYED configuration being **characterized by** the protrusion of said needles (20) out of said plurality of openings;
the disposition of said openings in said distal end (12) is provided according to a predetermined scattering pattern such that (i) at least two separated areas (30, 32) having more than three openings per area within said
distal end (12) are provided for simultaneous injection of said substance into at least two different injection locations (40, 42) at said injection site (44); and, (ii) a DEAD AREA (33) between said two separated areas (30, 32) is obtained, wherein at least one of the following being held true:
(i) said DEAD AREA (33) having a solid angle of at least at least about 1 steradian;
(ii) said DEAD AREA (33) having a spreading angle in the range of 20 to 60 degrees from each other;
(iii) said DEAD AREA (33) having a maximal length of at least 0.1 nanometer;
(iv) said DEAD AREA (33) having a geometrical area of at least 0.1 nanometer²;
(v) said DEAD AREA (33) having a cross sectional area of at least 0.1 nanometer²; wherein in said DEPLOYED configuration, said needles (20) are adapted to form at least two injection surfaces at said at least two separated areas (30, 32), each of said at least two injection surfaces is adapted to conform to the anatomical shape of said at least two injection locations (40, 42), the two injection locations (40, 42) being the upper lip (40) and the lower lip (42) of the uterine cervix (44) of female mammals, and wherein said needles are flexible.

2. The multi-directional needle assembly (100) according to claim 1, wherein at least one of the following is being held true (a) said at least two injection surfaces are adapted to mimic the anatomical shape of said at least two injection locations (40, 42) (b) each of said at least two injection surfaces is **characterized by** a predetermined spread angle ranging from about 100° to about 160° (c) each of said at least two injection surfaces is **characterized by** a predetermined spread angle ranging from 150° and 160°; and (d) said at least two injection surfaces are formed by different lengths of said needles (20).

3. The multi-directional needle assembly (100) according to claim 1, wherein at least one of the following is being held true (a) said predetermined scattering pattern is selected from a group consisting of: arbitrary, well organized, and any combination thereof; (b) said needles are microneedles; (c) said needles are 20 gauge to about 35 gauge needle; (d) at least one of said needles is one of a group of: nano-sized, micro-sized, milli-sized, or any combination thereof; (e) said needles are **characterized by** widths ranging from about 0.5 micron to about 400 micron; (f) said needles are **characterized by** widths ranging from about 100 nm to about 500 nm; (g) said substance is for induction of labor.

4. The multi-directional needle assembly (100) according to claim 1, further comprising at least one spreading mechanism adapted to reconfigure said needles (20) within said elongated member from a FOLDED configuration to a DEPLOYED configuration and vice versa; further wherein said spreading mechanism is adapted to protract said needles (20) when the same are converted from said FOLDED configuration to said DEPLOYED configuration, and to retract said needles (20) when the same are converted from said DEPLOYED configuration to said FOLDED configuration.

5. The multi-directional needle assembly (100) according to claim 1, wherein at least one of the following is being held true: (a) said substance is selected from a group consisting of a cervical-ripening amount of a collagenase or any naturally stimulating collagenase, Interleukin 1 beta, TNF or any combination thereof , configured to induce labor; (b) collagenase inhibitor, Interleukin 6, Interleukin 8, tissue inhibitors metalloproteinase 1-2, NAC n -acetyl cysteine TIMP tissue inhibitor metalloproteinases1-2, inhibition anti TNF, TNF antibodies anti IL 1 beta antibodies TIMP 1-2 Alfa 2 macroglobulin, alfa 2 macroglobulin IL-8 ETE IL 1beta antibodies TNF antibodies, collagen anti Illbeta alfa2 macroglobulin TIMP1-2 and any combination thereof configured to prevent and/or treat preterm labor.

6. The multi-directional needle assembly (100) according to claim 1, wherein at least one of the following is being held true (a) said needles (20) are made of a flexible material adapted to provide bending of said needles (20) when the same are reconfigured from said FOLDED configuration to said DEPLOYED configuration; (b) said elongated member (10) is selected from a group consisting of: a needle, a catheter, a lumen, or any combination thereof; (c) at least two of said needles (20) are aligned and oriented at substantially the same specific angle; (d) at least two of said needles (20) are aligned and oriented at different angles, said angles forming a predetermined pattern; (e) at least two of said needles (20) are randomly oriented; (f) at least two of said needles (20) are **characterized by** having the same length or thickness.

## Patentansprüche

1. Multidirektionale Nadelanordnung (100) zum Injizieren von Substanzen in mindestens eine Injektionsstelle am Körper eines Patienten, umfassend:
a. ein längliches Element (10) mit einem distalen Ende (12); wobei das distale Ende (12) eine Vielzahl darin befindlicher Öffnungen aufweist und
b. eine Vielzahl von Nadeln (20), die zumindest teilweise innerhalb des länglichen Elements (10) angeordnet sind, wobei die Nadeln (20) dazu ausgelegt sind, umkonfiguriert zu werden von einer GEFALTETEN Konfiguration in eine AUSGEFAHRENE Konfiguration und umgekehrt, wobei die GEFALTETE Konfiguration durch die Position der Nadeln (20) innerhalb des länglichen Elements (10) gekennzeichnet ist, wobei die AUSGEFAHRENE Konfiguration durch das Herausragen der Nadeln (20) aus den Öffnungen gekennzeichnet ist;
die Anordnung der Öffnungen in dem distalen Ende (12) folgt einem vorbestimmten Streumustert, sodass (i) mindestens zwei getrennte Bereiche (30, 32) mit mehr als drei Öffnungen pro Bereich innerhalb des distalen Endes (12) für die gleichzeitige Injektion der Substanzen in mindestens zwei verschiedene Injektionsstellen (40, 42) auf der Injektionsfläche (44) vorhanden sind; und (ii) ein TOTBEREICH (33) zwischen den beiden getrennten Bereichen (30, 32) erhalten wird, worin mindestens eine der folgenden Aussagen gilt:
(i) Der TOTBEREICH (33) weist einen Raumwinkel von mindestens ca. 1 sr auf,
(ii) der TOTBEREICH (33) weist einen Spreizwinkel zwischen 20 bis 60° voneinander auf,
(iii) der TOTBEREICH (33) weist eine maximale Länge von mindestens 0,1 nm auf,
(iv) der TOTBEREICH (33) weist eine geometrische Fläche von mindestens 0,1 nm² auf,
(v) der TOTBEREICH (33) weist eine Querschnittsfläche von mindestens 0,1 nm² auf, wobei in der AUSGEFAHRENEN Konfiguration die Nadeln (20) dazu ausgelegt sind, mindestens zwei Injektionsbereiche in den mindestens zwei getrennten Bereichen (30, 32) zu bilden, wobei jede der mindestens zwei Injektionsflächen dazu ausgelegt ist, sich an die anatomische Form der mindestens zwei Injektionsflächen (40, 42) anzupassen, wobei die beiden Injektionsflächen (40, 42) der innere Muttermund (40) und der äußere Muttermund (42) im Gebärmutterhalses (44) von weiblichen Säugetieren sind, und wobei die Nadeln flexibel sind.

2. Die multidirektionale Nadelanordnung (100) nach Anspruch 1, wobei mindestens eine der folgenden Eigenschaften gilt: (a) die mindestens zwei Einspritzflächen sind so angepasst, dass sie die anatomische Form der mindestens zwei Injektionsflächen (40, 42) nachahmen, (b) jede der mindestens zwei Einspritzflächen durch einen vorgegebenen Spreizwinkel im Bereich von etwa 100° bis etwa 160° gekennzeichnet ist, (c) jede der mindestens zwei Injektionsflächen durch einen vorgegebenen Spreizwinkel im Bereich von 150° bis 160° gekennzeichnet ist; und (d) die mindestens zwei Injektionsflächen durch unterschiedliche Längen der Nadeln (20) gebildet werden.

3. Die multidirektionale Nadelanordnung (100) nach Anspruch 1, wobei mindestens eine der folgenden Eigenschaften gilt: (a) das vorbestimmte Streumuster ist ausgewählt aus einer Gruppe bestehend aus: zufällig, klar organisiert und jede Kombination davon; (b) die Nadeln sind Mikronadeln; (c) die Nadeln haben 20 bis etwa 35 Gauge; (d) mindestens eine der Nadeln ist aus folgender Gruppe: Nanogröße, Mikrogröße, Milligröße oder jede Kombination davon; (e) die Nadeln sind **gekennzeichnet durch** Breiten im Bereich von etwa 0,5 µm bis etwa 400 µm; (f) die Nadeln sind haben Breiten im Bereich von etwa 100 nm bis etwa 500 nm; (g) die Substanz dient der Einleitung von Wehen.

4. Multidirektionale Nadelanordnung (100) nach Anspruch 1, die ferner mindestens einen Spreizmechanismus umfasst, der dazu ausgelegt ist, die Nadeln (20) innerhalb des langgestreckten Elements umzukonfigurieren von einer GEFALTETEN Konfiguration in eine AUSGEFAHRENE Konfiguration und umgekehrt; wobei ferner der Spreizmechanismus dazu ausgelegt ist, die Nadeln (20) zu verlängern, wenn sie von der GEFALTETEN Konfiguration in die AUSGEFAHRENE Konfiguration umgewandelt werden, und die Nadeln (20) zurückzufahren, wenn sie von der AUSGEFAHRENEN Konfiguration in die GEFALTETE Konfiguration konvertiert werden.

5. Die multidirektionale Nadelanordnung (100) nach Anspruch 1, wobei mindestens eine der folgenden Aussagen gilt: (a) die Substanz stammt ist aus einer Gruppe aus einer zervikal reifenden Menge einer Kollagenase oder einer natürlich stimulierenden Kollagenase, Interleukin-1β, TNF oder einer Kombination davon, die dazu ausgelegt ist, Wehen einzuleiten; (b) Kollagenase-Inhibitor, Interleukin-6, Interleukin-8, Gewebeinhibitoren Metalloproteinase 1-2, n-Acetylcystein (NAC) TIMP Gewebeinhibitor Metalloproteinasen 1-2, TNF-Hemmer, TNF-Antikörper gegen IL-1β-Antikörper TIMP 1-2, Alpha-2-Makroglobulin, Alpha-2-Makroglobulin IL-8 ETE IL 1β-Antikörper TNF-Antikörper, Kollagen Anti-Il1β ALpha-2-Makroglobulin TIMP1-2 und jede Kombination davon, die zur Verhinderung und/oder Behandlung von Frühgeburten ausgelegt ist.

6. Die multidirektionale Nadelanordnung (100) nach Anspruch 1, wobei mindestens eine der folgenden Aussagen gilt: (a) die Nadeln (20) sind aus einem flexiblen Material hergestellt, das dazu ausgelegt ist, ein Biegen der Nadeln (20) zu ermöglichen, wenn diese von der GEFALTETEN Konfiguration in die AUSGEFAHRENE Konfiguration umkonfiguriert werden; (b) das längliche Element (10) stammt aus einer Gruppe bestehend aus: eine Nadel, ein Katheter, ein Lumen oder eine beliebige Kombination davon; (c) mindestens zwei der Nadeln (20) sind ausgerichtet und im Wesentlichen im gleichen spezifischen Winkel orientiert; (d) mindestens zwei der Nadeln (20) sind ausgerichtet und in verschiedenen Winkeln orientiert, wobei die Winkel ein vorgegebenes Muster bilden; (e) mindestens zwei der Nadeln (20) sind zufällig ausgerichtet; (f) mindestens zwei der Nadeln (20) haben die gleiche Länge oder Dicke.

## Revendications

1. Assemblage d'aiguilles multidirectionnelles (100) destinées à l'injection d'une substance dans au moins un site d'injection du corps d'un patient, comprenant :
a. un élément allongé (10) possédant une extrémité distale (12), un certain nombre d'ouvertures étant disposées dans ladite extrémité distale (12) ; et
b. un certain nombre d'aiguilles (20) qui peuvent venir se disposer au moins en partie au sein dudit élément allongé (10), lesdites aiguilles (20) étant conçues pour être soumises à une reconfiguration à partir d'une configuration REPLIEE jusque dans une configuration DEPLOYEE et vice versa, ladite configuration REPLIEE étant **caractérisée par** la position desdites plusieurs aiguilles (20) au sein dudit élément allongé (10) ; ladite configuration DEPLOYEE étant **caractérisée par** la protrusion desdites aiguilles (20) à l'extérieur desdites plusieurs ouvertures ;
la disposition desdites ouvertures dans ladite extrémité distale (12) est prévue en conformité avec un modèle de dispersion prédéterminé d'une manière telle que : (i) on prévoit au moins deux zones séparées (30, 32) possédant plus de trois ouvertures par zone au sein de ladite extrémité distale (12) pour une injection simultanée de ladite substance dans au moins deux endroits d'injection différents (40, 42) audit site d'injection (44) ; et (ii) on obtient une ZONE MORTE (33) entre lesdites deux zones séparées (30, 32) ; dans lequel au moins une des affirmations suivantes est vraie, à savoir :
(i) lesdites ZONES MORTES (33) possèdent un angle solide d'au moins environ 1 stéradian ;
(ii) lesdites ZONES MORTES (33) possèdent un angle d'expansion dans la plage de 20 à 60° l'une par rapport à l'autre ;
(iii) lesdites ZONES MORTES (33) possèdent une longueur maximale d'au moins 0,1 nm ;
(iv) lesdites ZONES MORTES (33) possèdent une aire géométrique d'au moins 0,1 nm² ;
(v) lesdites ZONES MORTES (33) possèdent une aire en coupe transversale d'au moins 0,1 nm² ;
dans lequel, dans ladite configuration DEPLOYEE, lesdites aiguilles (20) sont conçues pour former au moins deux surfaces d'injection dans lesdites au moins deux zones séparées (30, 32), chacune desdites au moins deux surfaces d'injection est conçue pour épouser la configuration anatomique desdits au moins deux endroits d'injection (40, 42), les deux endroits d'injection (40, 42) représentent la lèvre supérieure (40) et la lèvre inférieure (42) du col de l'utérus (44) de mammifères femelles ; et dans lequel lesdites aiguilles sont flexibles.

2. Assemblage d'aiguilles multidirectionnelles (100) selon la revendication 1, dans lequel au moins une des affirmations suivantes est vraie, à savoir : (a) lesdites au moins deux surfaces d'injection sont conçues pour épouser la configuration anatomique desdits au moins deux endroits d'injection (40, 42) ; (b) chacune desdites au moins deux surfaces d'injection est **caractérisée par** un angle d'expansion prédéterminé qui se situe dans la plage d'environ 100° à environ 160° ; (c) chacune desdites au moins deux surfaces d'injection est **caractérisée par** un angle d'expansion prédéterminé qui se situe dans la plage de 150° à 160° ; et (d) lesdites au moins deux surfaces d'injection sont formées par différentes longueurs desdites aiguilles (20).

3. Assemblage d'aiguilles multidirectionnelles (100) selon la revendication 1, dans lequel au moins une des affirmations suivantes est vraie, à savoir : (a) ledit modèle de diffusion prédéterminé est choisi parmi un groupe constitué par : un modèle arbitraire, un modèle bien organisé et l'une quelconque de leurs combinaisons ; (b) lesdites aiguilles représentent des micro-aiguilles ; (c) lesdites aiguilles représentent des aiguilles de calibres 20 à environ 35 ; (d) au moins une desdites aiguilles représente une aiguille choisie parmi un groupe comprenant : une aiguille de dimension nanométrique, une aiguille de dimension microscopique, une aiguille de dimension millimétrique, ou l'une quelconque de leurs combinaisons ; (e) lesdites aiguilles sont **caractérisées par** des largeurs qui se situent dans la plage allant d'environ 0,5 micron à environ 400 microns ; (f) lesdites aiguilles sont **caractérisées par** des largeurs qui se situent dans la plage allant d'environ 100 nm à environ 500 nm ; (g) ladite substance est destinée à induire le travail lors d'un accouchement.

4. Assemblage d'aiguilles multidirectionnelles (100) selon la revendication 1, comprenant en outre au moins un mécanisme d'expansion conçu pour reconfigurer lesdites aiguilles (20) au sein dudit élément allongé à partir d'une configuration REPLIEE jusque dans une configuration DEPLOYEE et vice versa ; en outre, dans lequel ledit mécanisme d'expansion est conçu pour étendre lesdites aiguilles (20) lorsque ces dernières font l'objet d'une conversion en passant de ladite configuration REPLIEE à ladite configuration DEPLOYEE et pour rétracter lesdites aiguilles (20) lorsque ces dernières font l'objet d'une conversion en passant de ladite configuration DEPLOYEE à ladite configuration REPLIEE.

5. Assemblage d'aiguilles multidirectionnelles (100) selon la revendication 1, dans lequel au moins une des affirmations suivantes est vraie, à savoir que ladite substance est choisie parmi un groupe constitué par : (a) une quantité d'une collagénase ou de n'importe quelle collagénase ayant un effet de stimulation naturel, de l'interleukine 1 bêta, du TNF ou de l'une quelconque de leurs combinaisons, la quantité en question donnant lieu à une maturation du col et ladite substance étant configurée pour induire le travail lors de l'accouchement ; (b) une substance qui est choisie parmi un groupe constitué par : un inhibiteur de la collagénase, l'interleukine 6, l'interleukine 8, les métalloprotéinases 1-2 à titre d'inhibiteurs tissulaires, la n-acétyl cystéine NAC, des métalloprotéinases 1-2 TIMP à titre d'inhibiteurs tissulaires, des inhibiteurs anti TNF, des anticorps anti TNF anti IL 1 bêta, des anticorps anti TIMP 1-2, la macroglobuline Alfa 2, la macroglobuline alfa 2, des anticorps anti IL 1 bêta IL-8 ETE, des anticorps anti TNF, du collagène anti IL 1 bêta, la macroglobuline alfa 2, TIMP 1-2, ainsi que l'une quelconque de leurs combinaisons, ladite substance étant configurée pour empêcher et/ou pour traiter un travail prématuré lors de l'accouchement.

6. Assemblage d'aiguilles multidirectionnelles (100) selon la revendication 1, dans lequel au moins une des affirmations suivantes est vraie, à savoir : (a) lesdites aiguilles (20) sont réalisées à partir d'une matière flexible conçue pour procurer une flexion desdites aiguilles (20) lorsque ces dernières sont soumises à une reconfiguration à partir de ladite configuration REPLIEE jusque dans ladite configuration DEPLOYEE ; (b) ledit élément allongé (10) est choisi parmi un groupe constitué par : une aiguille, un cathéter, une lumière, ou l'une quelconque de leurs combinaisons ; (c) au moins deux desdites aiguilles (20) sont alignées et sont orientées en formant essentiellement le même angle spécifique ; (d) au moins deux desdites aiguilles (20) sont alignées et sont orientées en formant des angles différents ; (e) au moins deux desdites aiguilles (20) sont orientées d'une manière aléatoire ; (f) au moins deux desdites aiguilles (20) sont **caractérisées par** le fait de posséder la même longueur ou la même épaisseur.
